# EUROPEAN PATENT SPECIFICATION

(11) **EP 2 813 218 B1**
(45) Date of publication and mention of the grant of the patent: **24.08.2022**
(21) Application number: 14169540.3
(22) Date of filing: 22.05.2014
(51) Int. Cl.: A61K 9/28, A61K 31/525

(54) **METHOD FOR PRODUCING AQUEOUS ENTERIC COATING LIQUID, SOLID PREPARATION, AND METHOD FOR PRODUCING SAME**
VERFAHREN ZUR HERSTELLUNG EINER WÄSSRIGEN ENTERISCHEN BESCHICHTUNGSFLÜSSIGKEIT, FESTSTOFFPRÄPARAT UND VERFAHREN ZUR HERSTELLUNG DAVON
PROCÉDÉ DE PRODUCTION DE LIQUIDE D'ENROBAGE ENTÉROSOLUBLE AQUEUX, PRÉPARATION SOLIDE ET SON PROCÉDÉ DE PRODUCTION

(30) Priority: 14.06.2013 JP 2013125543
(43) Date of publication of application: 17.12.2014
(73) Proprietor: Shin-Etsu Chemical Co., Ltd., Chiyoda-ku, Tokyo (JP)
(72) Inventor: Kikuchi, Shingo, Joetsu-shi, Niigata (JP); Hoshino, Takafumi, Joetsu-shi, Niigata (JP); Nishiyama, Yuichi, Joetshu-shi, Niigata (JP)
(74) Representative: HGF

(56) References cited:
- EP-A1- 1 356 825
- WO-A1-2005/026233
- WO-A2-2010/080970
- US-A- 5 225 202

## Description

### BACKGROUND OF THE INVENTION

### 1. Field of the invention

The present invention relates to a method for producing an aqueous enteric coating liquid, and a method for producing the solid preparation.

### 2. Description of the related art

A method of coating an enteric material dissolved in an organic solvent has had problems such as risk of explosion or fire of the organic solvent used in a large amount, environmental contamination with the organic solvent released into the air, and toxicity of the organic solvent remaining in a pharmaceutical. The method also has the disadvantage of requiring introduction of a solvent recovery unit.

In aqueous dispersion type coating developed in order to overcome such problems, an aqueous dispersion of an enteric material is sprayed as a mist to maintain the powdery enteric material, which is allowed to be discontinuously present on the sprayed surface of an object. After spraying, as the dispersion attached to the surface of the object becomes dry, the plasticizer sprayed simultaneously penetrates into the enteric material particles and plasticizes the enteric material to form an enteric film. The required coating amount varies, depending on the shape of the object to be coated, or properties of the drug or additive contained by the coating such as solubility in water.

In particular, for dispersing a cellulosic enteric polymer in water, a method for neutralizing the carboxyl group of the polymer (Japanese Patent Application Unexamined Publication No. 56-030913), and a method for dispersing a cellulose-type enteric polymer, which has been ground into fine particles, in water comprising a high boiling-point gelling agent such as triethyl citrate (Japanese Patent Application Examined Publication Nos. 57-053329 and 58-055125) are widely known. On the other hand, an aqueous enteric coating liquid obtained by partially neutralizing an enteric polymer with an aqueous ammonia solution is also disclosed (Japanese Patent Application Unexamined Publication Nos. 61-263930, 8-245423 and 8-040895).

EP1356825 A1 describes a process for producing a cellulose derivative aqueous dispersion.

### SUMMARY OF THE INVENTION

In the method of Japanese Patent Application Unexamined Publication No. 56-030913, however, an alkali salt or ammonium salt of carboxylic acid produced by the neutralization remains in the coating film of a solid enteric preparation. The alkali salt or ammonium salt has high hygroscopicity and tends to be readily soluble in water so that the solid enteric preparation thus obtained has a deteriorated quality. In addition, the neutralization markedly increases the viscosity of the coating liquid so that the applicable ranges of a coating concentration and a spraying rate are narrowed, leading to reduced production efficiency. In the methods of Japanese Patent Application Examined Publication Nos. 57-053329 and 58-055125, in order to prevent clogging of a nozzle which will otherwise occur by the gelation of a heat-sensitive aqueous dispersion during coating, an apparatus for cooling the aqueous dispersion as well as a tube and a nozzle becomes indispensable. In particular, when hydroxypropylmethyl cellulose acetate succinate (which may hereinafter be called "HPMCAS") is used as an enteric material, cooling of the aqueous dispersion to 10°C or lower prior to coating, as well as cooling of the tube and the nozzle, is required. There is therefore a demand for the development of a coating method as simple as possible.

In the method of Japanese Patent Application Unexamined Publication No. 61-263930, a cellulosic enteric material, a plasticizer and a film-forming aid are mixed simultaneously so that in some combinations, the enteric material and the plasticizer form an aggregate. On the other hand, in Japanese Patent Application Unexamined Publication No. 8-245423, the degree of neutralization is 80 mol% or higher and such an excessively high degree of neutralization increases the concentration of the coating liquid and requires a large amount of coating. Further, although Japanese Patent Application Unexamined Publication No. 8-040895 specifies that the degree of neutralization is from about 25 to 100 mol%, a cooling equipment for keeping the liquid temperature constant during cooling at the time of preparation of the coating liquid and during coating is necessary unless complete neutralization having the degree of neutralization of 100 mol% is achieved.

With a view to overcoming the above-mentioned problems, the invention has been made. An object is to easily provide, by using a simple and efficient method without a special cooling equipment, an enteric preparation having a film-forming property and acid resistance which are equal to or more excellent than those of the enteric coating preparation obtained by a conventional aqueous enteric coating method.

As a result of making an extensive investigation to achieve the above-mentioned object, the present inventors have found that the above-mentioned object is achieved only when an aqueous suspension of an enteric material is partially neutralized with an aqueous alkali solution such as an aqueous ammonia solution, and a coating liquid is prepared in a predetermined order, thereby leading to completion of the invention.

According to the invention, there is provided a method for producing an aqueous enteric coating liquid comprising the steps of: (i) partially neutralizing an aqueous suspension comprising a cellulosic enteric material with an aqueous alkali solution, and (ii) mixing the partially neutralized aqueous suspension with a plasticizer, wherein a degree of neutralization with the aqueous alkali solution is from 5 to 25 mol% of the carboxy groups of the cellulosic enteric material, and wherein the cellulosic enteric material is a cellulose ester containing a carboxy group for preventing dissolution in an acidic liquid.

There is further provided a method for producing a solid preparation comprising: respective steps in the method for producing an aqueous enteric coating liquid, and a step of coating a drug-comprising core with the produced aqueous enteric coating liquid.

Use of the aqueous enteric coating liquid obtained by the production method in accordance with the invention makes it possible to provide an enteric coating preparation capable of forming a dense film and securing excellent acid resistance in a smaller coating amount compared with a conventional method. In addition, an organic solvent is not necessary because it is an aqueous system. Further, it is thermally stable so that a special apparatus for temperature control such as for cooling is not necessary and a conventional coating equipment or technology can be used as it is. Still further, coating is conducted at a low degree of neutralization so that the viscosity of the coating liquid is low, the concentration of a cellulosic enteric material can be made drastically greater than that in the conventional method, and as a result, a reduction in production cost and efficient production can be achieved.

### DETAILED DESCRIPTION OF THE PREFERRED EMBODIMENTS

The method for producing an aqueous enteric coating liquid will hereinafter be described.

According to the invention, the aqueous enteric coating liquid is obtained by partially neutralizing a cellulosic enteric material with an aqueous alkali solution and then adding a plasticizer to the resulting aqueous suspension.

The cellulosic enteric material is a polymer having a dissolution level of "practically insoluble, or insoluble (the volume of water required for dissolving 1 g or 1 ml of a drug is 10000 mL and over)" under the conditions specified in the Japanese Pharmacopoeia 16th Edition and being soluble in an alkaline solution. Specific Examples of the cellulosic enteric material include cellulose acetate phthalate, cellulose acetate trimellitate, cellulose acetate succinate, methyl cellulose phthalate, hydroxymethylcellulose ethyl phthalate, hydroxypropylmethyl cellulose phthalate (HPMCP), hydroxypropylmethyl cellulose acetate succinate (HPMCAS), hydroxypropylmethyl cellulose acetate maleate, hydroxypropylmethyl cellulose trimellitate.

Of these, hydroxypropylmethyl cellulose phthalate (HPMCP) and hydroxypropylmethyl cellulose acetate succinate (HPMCAS) are preferred because they are relatively free of an aggregation phenomenon and achieve a high production yield.

Among HPMCAS, particularly HPMCAS having a property presumed to start drug release promptly after being transferred from the stomach to the upper or middle portion of the small intestine is preferred. More specifically, HPMCAS having a property of being dissolved within 120 minutes in a Japanese Pharmacopoeia phosphate buffer solution having a pH value of from 5 to 7 (5.0 to 6.8) is preferred.

As for the factors of exhibiting the solubility of HPMCAS polymer, an amount of each substituent and an acetyl to succinoyl composition ratio can be considered. Specific examples include, but not limited to, the following:

Specific Example 1: methoxyl group of from 20 to 24% by weight, hydroxypropoxyl group of from 5 to 9% by weight, acetyl group of from 5 to 9% by weight, and succinoyl group of from 14 to 18 by weight% such as grade "AS-L" produced by Shin-Etsu Chemical Co., Ltd.;

Specific Example 2: methoxyl group of from 21 to 25% by weight, hydroxypropoxyl group of from 5 to 9% by weight, acetyl group of from 7 to 11% by weight, and succinoyl group of from 10 to 14% by weight such as grade "AS-M" produced by Shin-Etsu Chemical Co., Ltd.; and

Specific Example 3: methoxyl group of from 22 to 26 by weight, hydroxypropoxyl group of from 6 to 10% by weight, acetyl group of from 10 to 14% by weight, and succinyl group of from 4 to 8% by weight such as grade "AS-H" produced by Shin-Etsu Chemical Co., Ltd.

The HPMCAS having a substituent content other than the above-mentioned contents can be used if it exhibits a pH dissolution of within 120 minutes in a phosphate buffer having a pH value of from 5 to 7 (from 5.0 to 6.8) when it is combined with any HPMCAS of Specific Examples 1 to 3.

The cellulosic enteric material is a cellulose ester containing a carboxy group for preventing dissolution in an acidic liquid. The amount of the carboxy group of the cellulosic enteric material is, for example, preferably from 4 to 28% by weight for HPMCAS and preferably from 21 to 35% by weight for HPMCP. The amount of the carboxy groups of the cellulosic enteric material can be measured using a determination method described in Hypromellose Acetate Succinate (HPMCAS) and Hypromellose Phthalate (HPMCP) in the Japanese Pharmacopoeia 16th Edition.

The aqueous suspension comprising the cellulosic enteric material can be prepared by adding the cellulosic enteric material to purified water and dispersing the resulting mixture by stirring with a propeller stirrer or homogenizer, which is also used in the partial neutralization step which will be described later. During preparation of the aqueous suspension, relatively mild stirring of the suspension is maintained so as to prevent agglomeration of the polymer due to foaming. The preferable number of revolutions is from 100 to 1200 rpm for a propeller stirrer and from 500 to 10000 rpm for a homogenizer.

The dispersion stability of the enteric material may be improved by adding in advance (prior to the addition of the cellulosic enteric material) an optional (anionic) surfactant including sodium alkyl sulfate such as sodium lauryl sulfate or dioctyl sodium sulfosuccinate, and fatty acid sodium or potassium such as sodium oleate or potassium sorbate. The surfactant is added in an amount of preferably from 0.1 to 10 parts by weight, especially preferably from 1.0 to 5.0 parts by weight, each based on 100 parts by weight of the cellulosic enteric material, from the standpoint of dispersibility and water resistance.

Then, an aqueous alkali solution in an amount necessary for partially neutralizing the cellulosic enteric material is added to the aqueous suspension. The resulting mixture is stirred preferably for about 30 minutes to sufficiently react the cellulosic enteric material with the alkali.

Examples of the aqueous alkali solution include respective aqueous solutions of ammonia, monoethanolamine and sodium hydroxide. In particular, the aqueous ammonia solution is preferred because ammonia is removed by vaporization during drying in a coating operation and almost no alkali salt remains.

According to the invention, the degree of neutralization of the cellulosic enteric material by using the aqueous alkali solution is from 5 to 25 mol%, preferably from 10 to 20 mol%, still more preferably from 15 to 20 mol%, each based on the carboxy groups of the cellulosic enteric material. When the degrees of neutralization is less than 5 mol%, sufficient film formation may be prevented because of the insufficient film-forming property. When the degree of neutralization is more than 25 mol%, the viscosity of the suspension may show a marked increase as the degree of neutralization increases, or the remaining alkali salt may enhance a water-conducting property to prevent sufficient acid resistance from being secured. In addition, the viscosity of the coating liquid thus prepared also depends on the concentration of the enteric material dissolved therein so that an increase in the degree of neutralization may limit the concentration of the coating liquid that enables a coating operation.

According to the invention, examples of the plasticizer include citric acid esters such as triethyl citrate and acetylated triethyl citrate; and glycerin fatty acid esters such as triacetin, polyethylene glycol, propylene glycol, glycerin and monoacetyl glycerin. The plasticizer is used in an amount of preferably from 6 to 28 parts by weight, more preferably from 10 to 20 parts by weight, still more preferably from 15 to 20 parts by weight, each based on 100 parts by weight of the cellulosic enteric material. When an amount of the plasticizer is less than 6 parts by weight, the film-forming property may be deteriorated. When an amount of the plasticizer is more than 28 parts by weight, the economic efficiency may be deteriorated.

In order to prevent clogging of a nozzle which will otherwise occur by the gelation of the aqueous enteric coating liquid during coating, it is necessary to completely dissolve the plasticizer and prevent precipitation due to a temperature increase during coating. In a conventional aqueous dispersion system, when a plasticizer is dissolved in water at room temperature in advance, an aqueous alkali solution is added thereto, and then a cellulosic enteric material is dispersed in the resulting mixture. In another conventional aqueous dispersion system, a plasticizer is suspended in water in advance at room temperature, a cellulosic enteric material is dispersed therein, and then the resulting dispersion is partially neutralized with an aqueous alkali solution. In these conventional aqueous dispersion systems, even a slight amount of the undissolved plasticizer which has remained without being fully dissolved may deteriorate the dispersibility of the cellulosic enteric material and form an aggregate at room temperature. In order to prevent formation of an aggregate, it is necessary to sufficiently dissolve the plasticizer in water cooled in advance and to disperse the cellulosic enteric material in the resulting solution, and moreover, to cool the tube and nozzle of a coating apparatus so as to prevent precipitation of the plasticizer due to a temperature increase of the aqueous enteric coating liquid. However, in the method in accordance to the invention, partial neutralization of the cellulosic enteric material with an aqueous alkali solution allows a portion of the cellulosic enteric material to become a water-soluble ammonium salt, having a surface active action and an electrostatic interaction for lowering the aggregation between particles. Accordingly, an aqueous enteric coating liquid can be produced without special equipment such as a cooling apparatus.

According to the invention, an optional excipient can be mixed for adhesion prevention or coloring after, before or during inclusion of the plasticizer into the partially neutralized aqueous suspension. Examples of the excipient include talc, titanium oxide and silicon dioxide. Talc is particularly preferred from the standpoint of preventing adhesion between particles during coating. The excipient is added in an amount of preferably 100 parts by weight or less, more preferably 50 parts by weight or less, each based on 100 parts by weight of the cellulosic enteric material. When the amount of the excipient is more than 100 parts by weight, dusting of a spray mist may take place.

According to the invention, the aqueous enteric coating liquid may optionally comprise a lubricant, a colorant, a pigment, a sweetener, a defoaming agent, or the like. It may also be mixed after, before or during inclusion of the plasticizer into the partially neutralized aqueous suspension.

The concentration of the enteric material in the aqueous enteric coating liquid can be increased or decreased unless it does not adversely affect the coating operation and coating conditions. For example, when hydroxypropylmethyl cellulose acetate succinate (HPMCAS) is used as an enteric material, the concentration in accordance with to the invention is preferably from 3 to 20% by weight, more preferably from 7 to 20% by weight, still more preferably from 10 to 18% by weigh, contrary to the conventional concentration of 7% by weight or less in a conventional aqueous dispersion system in which the aggregation temperature of the plasticizer and the material decreases as the material concentration increases. When the concentration is less than 3% by weight, productivity may be deteriorated because a markedly long coating time becomes necessary. When the concentration is more than 20% by weight, the viscosity may increase and therefore may deteriorate coating operability.

The viscosity of the aqueous enteric coating liquid is not particularly limited insofar as it falls within a range permitting spraying for coating. Typically, the viscosity at 20°C of the aqueous enteric coating liquid is preferably from 1 to 200 mPa·s, more preferably 100 mPa·s or less, particularly preferably 50 mPa·s or less. When the viscosity is more than 200 mPa·s, transferring the coating liquid to a pump or spray gun may become difficult and therefore coating operability may be deteriorated. The viscosity can be determined according to the viscosity measurement method described in the Japanese Pharmacopoeia 16th Edition.

Next, a solid preparation will be described.

The solid preparation comprises a core comprising a drug, and a coating portion obtained by coating the core with the above-mentioned aqueous enteric coating liquid.

The drug which can be present in the drug-comprising core is not particularly limited insofar as it is a pharmacologically active component recognized as a component for medicinal use. Examples of such a drug include chemotherapeutic agents; respiratory stimulants; anti-malignant tumor agents; autonomic agents; psychoneurotic agents; local anesthetics; muscle relaxants; pharmaceutical agents for digestive organs; drugs for poisoning treatment; hypnotic sedatives; vasodilators; antilipemic agents; nutritional additives for medical purposes, medicinal tonics and substitutes therefor; anticoagulants; pharmaceutical agents for liver; hypoglycemic agents; antihypertensives; anticolitic drugs; peptides; proteins; antibiotics such as talampicillin hydrochloride, bacampicillin hydrochloride, cefaclor and erythromycin; antitussive and expectorant drugs such as noscapine hydrochloride, carbetapentane citrate, dextromethorphan hydrobromide, isoaminile citrate and dimemorfan phosphate; antihistamine drugs such as chlorpheniramine maleate, diphenhydramine hydrochloride and promethazine hydrochloride; antipyretic, analgesic and anti-inflammatory drugs such as ibuprofen, diclofenac sodium, flufenamic acid, sulpyrine, aspirin and ketoprofen; cardiotonics such as etilefrine hydrochloride and digitoxin; antiarrhythmic agents such as propranolol hydrochloride and alprenolol hydrochloride; diuretics such as caffeine; bronchodilators such as theophylline; anti-ulcer drugs such as cimetidine and pirenzepine hydrochloride; sympathetic stimulants such as dihydrocodeine phosphate and dl-methylephedrine hydrochloride; cardiovascular agents such as delapril hydrochloride, meclofenoxate hydrochloride and diltiazem hydrochloride; cerebral circulation activators such as vinpocetine; anxiolytics such as chlordiazepoxide and diazepam; vitamin preparations such as fursultiamine, thiamine hydrochloride, calcium pantothenate, ascorbic acid and tranexamic acid; antimalarial drugs such as quinine hydrochloride; antidiarrheal drugs such as loperamide hydrochloride; psychotropic drugs such as chlorpromazine; and vitamins such as Vitamin A, Vitamin B1, Vitamin B2, Vitamin B6, Vitamin B 12, Vitamin C, Vitamin D, Vitamin E and Vitamin K).

The drug may be used singly or in combination of two or more drugs.

The drug-comprising core may be a drug substance; a granulated particle obtained by wet granulation, dry granulation or the like; a layered particle containing a drug coating or drug layer around a nucleus, which is, for example, a nucleus of crystalline cellulose, mannitol or lactose; or the like. During formation of the drug-containing core by granulation or layering, a various additive typically used in this field such as excipient, binder, disintegrant or the like may be added.

An amount of the enteric material to be coated on the surface of the core may differ depending on the kind, shape, size, or surface condition of the core, or the property of the drug or additive contained in the core. In general, a coating amount for a tablet may be preferably from 3 to 100% by weight, more preferably from 6 to 12% by weight, and a coating amount for fine granules or granules may be preferably from 3 to 100 % by weight, more preferably from 15 to 30% by weight, each in terms of a total coating weight of the enteric material in the aqueous enteric coating liquid, relatively to the core weight. When the coating amount is less than 3% by weight, a continuous film having sufficient acid resistance may not be obtained. When the coating amount is more than 100% by weight, an enormous amount of coating time may be required and result in poor productivity.

The coating portion may be a coating portion or layer obtained from application of the aqueous enteric coating liquid only. An undercoat comprising another coating material may be present under the coating portion. The another coating material can include a various coating material typically used in this field such as hydroxypropylmethyl cellulose. The form of the coating portion is not particularly limited and may include a layered form and a film form.

Examples of the solid preparation include a tablet, a granule, a fine granule, and a capsule. The solid preparation may also include an orally disintegrating tablet. The solid preparation may comprise, in addition to a drug-containing particle, a various additive typically used in this field such as an excipient, a binder, a disintegrant, a lubricant, an anti-aggregation agent, or a solubilizing agent for a pharmaceutical compound. The additive may be present in any of the core of the solid preparation, the coating portion obtained by coating the aqueous enteric coating liquid, and the undercoat layer formed by undercoating, depending on its intended use.

Examples of the excipient include sugars such as sucrose, lactose and glucose; sugar alcohols such as mannitol, sorbitol and erythritol; starches; crystalline cellulose; calcium phosphate; and calcium sulfate.

Examples of the binder include polyvinyl alcohol, polyacrylic acid, polymethacrylic acid, polyvinylpyrrolidone, glucose, sucrose, lactose, maltose, dextrin, sorbitol, mannitol, hydroxyethyl cellulose, hydroxypropylmethyl cellulose, hydroxypropyl cellulose, macrogols, gum arabic, gelatin, agar and starches.

Examples of the disintegrant include low-substituted hydroxypropyl cellulose, carmellose and salt thereof, croscarmellose sodium, carboxymethyl starch sodium, crospolyvinylpyrrolidone, crystalline cellulose, and crystalline cellulose·carmellose sodium.

Examples of the lubricant and the anti-aggregation agent include talc, magnesium stearate, calcium stearate, colloidal silica, stearic acid, waxes, hydrogenated oils, polyethylene glycols, and sodium benzoate.

Examples of the solubilizing agent for a pharmaceutical compound include an organic acid such as fumaric acid, succinic acid, malic acid and adipic acid.

A method for producing the solid preparation will be described.

The solid preparation can be produced by coating the drug-comprising core with the aqueous enteric coating liquid by using a conventionally known coating apparatus.

Examples of the coating method include a method for applying the aqueous enteric coating liquid to the drug-comprising core.

It is also possible to form two or more of films by adding a step of forming undercoat between the drug-comprising core and a film layer formed by the application of the aqueous enteric coating liquid, through application of a various coating material typically used in this field such as hydroxypropylmethyl cellulose.

The coating apparatus is not particularly limited and can include a pan coating apparatus, a fluidized bed granulator, and a tumbling fluidized bed coating apparatus.

### EXAMPLES

The present invention will hereinafter be described specifically by Examples and Comparative Examples.

### <Example 1>

After 3 g (3 parts by weight based on 100 parts by weight of HPMCAS) of sodium lauryl sulfate was dissolved in 843.3 g of purified water at room temperature, 100 g of HPMCAS (grade AS-MF produced by Shin-Etsu Chemical Co., Ltd. ) was dispersed therein, while stirring with a propeller type stirrer to prepare an aqueous suspension of the HPMCAS. While stirring with the propeller type stirrer, an aqueous 10% by weight ammonia solution was added to the aqueous suspension to neutralize 20 mol% of the carboxy groups of the HPMCAS. After stirring for further 30 minutes, 20 g (20 parts by weight based on 100 parts by weight of the HPMCAS) of triethyl citrate and 30 g (30 parts by weight based on 100 parts by weight of the HPMCAS) of talc (Crown Talc produced by Matsumura Sangyo Co., Ltd.) were added. The resulting mixture was stirred for 10 minutes to prepare an aqueous enteric coating liquid having an HPMCAS concentration of 10% by weight. The aqueous enteric coating liquid was filtered through a 100-mesh sieve. No aggregate was found even by heating to 45°C.

Uncoated tablets were produced by mixing 2 parts by weight of riboflavin (product of Tokyo Tanabe Pharma Corporation), 90 parts by weight of lactose (Dilactose S produced by Freund Corporation), 8 parts by weight of low-substituted hydroxypropyl cellulose (hydroxypropyl substitution: 11% by weight), and 0.5 part of magnesium stearate, and tableting the resulting mixture into tablets at a tableting pressure of 1 t, a tableting pre-pressure of 0.3 t, and a rotation speed of 20 rpm by using a rotary tableting machine (Vergo produced by Kikusui Seisakujo Ltd.) to allow each tablet to have a diameter of 8 mm and a weight of 200 mg.

The 100 parts by weight of the uncoated tablets was coated with 6 parts by weight, in terms of a solid content weight, of the enteric aqueous coating liquid produced above, under the conditions below. After completion of the coating, drying and polishing were performed simultaneously in the same coating apparatus at a supply air temperature of 60°C and a rotation speed of the pan of 18 rpm for 30 minutes to obtain intended coated tablets.
Apparatus: vented pan coater having an inner diameter of 33 cm,
Charged amount: 1 kg,
Intake air temperature: 70°C,
Exhaust gas temperature: from 40 to 42°C,
Intake air flow rate: 1 m³/min,
Rotation speed of a pan: from 18 to 24 rpm,
Spray rate: from 5 to 10 g/min, and
Spray air pressure: 150 kPa

The disintegration test of 20 coated tablets thus obtained was conducted in accordance with the Japanese Pharmacopoeia by using 900ml of the First Liquid (pH 1.2) for disintegration test described in the Japanese Pharmacopoeia 16th Edition. The First liquid of the Japanese Pharmacopoeia corresponds to an artificial gastric fluid and is used for evaluation of acid resistance to a gastric fluid. A tablet loss ratio two hours after the disintegration test and a First Liquid permeability determined based on tablet weights before and after the disintegration test were measured for the acid resistance. The coated tablets were evaluated as defining a tablet having a tablet loss ratio of 0% and a First Liquid permeability of not greater than 5% as a tablet having sufficient acid resistance.

### <Comparative Example 1>

After 3 g (3 parts by weight based on 100 parts by weight of HPMCAS) of sodium lauryl sulfate was dissolved in 843.3 g of purified water at room temperature, 20 g (20 parts by weight based on 100 parts by weight of the HPMCAS) of triethyl citrate was added thereto, while stirring with a propeller type stirrer. Then, 100 g of HPMCAS (grade "AS-MF" produced by Shin-Etsu Chemical Co., Ltd.) was dispersed therein, and an aqueous 10% by weight ammonia solution was added to the resulting dispersion to neutralize a 20 mol% of the carboxy groups of the HPMCAS. After stirring for further 30 minutes with a propeller type stirrer, 30 g (30 parts by weight based on 100 parts by weight of the HPMCAS) of talc (Crown Talc produced by Matsumura Corporation) was added thereto. The resulting mixture was stirred for 10 minutes to produce an aqueous enteric coating liquid. When the aqueous enteric coating liquid was filtered through a 100-mesh sieve, aggregation was found at room temperature. The aggregate appeared because the HPMCAS dissolved in triethyl citrate which had not been dissolved at room temperature, caused adhesion of the HPMCAS particles.

### <Example 2>

Coated tablets were obtained in the same manner as in Example 1 except for use of the aqueous enteric coating liquid produced by changing the degree of neutralization of the carboxy groups of the HPMCAS to 15 mol % by using an aqueous 10% by weight ammonia solution.

### <Example 3>

Coated tablets were obtained in the same manner as in Example 1 except for use of the aqueous enteric coating liquid produced by changing the degree of neutralization of the carboxy groups of the HPMCAS to 5 mol% by using an aqueous 10% by weight ammonia solution.

### <Example 4>

Coated tablets were obtained in the same manner as in Example 1 except for use of the aqueous enteric coating liquid produced by changing the degree of neutralization of the carboxy groups of the HPMCAS to 25 mol% by using an aqueous 10% by weight ammonia solution.

### <Example 5>

Coated tablets were obtained in the same manner as in Example 1 except for use of the aqueous enteric coating liquid produced by using, as the neutralizer of the HPMCAS, an aqueous 17% by weight sodium hydroxide solution instead of the aqueous ammonia solution.

### <Example 6>

Coated tablets were obtained in the same manner as in Example 1 except for use of the aqueous enteric coating liquid produced by using, as the neutralizer of the HPMCAS, monoethanolamine instead of the aqueous ammonia solution.

### <Example 7>

Coated tablets were obtained in the same manner as in Example 1 except for use of the aqueous enteric coating liquid produced by using, as the plasticizer, 20 g (20 parts by weight based on 100 parts by weight of the HPMCAS) of triacetin instead of 20 g of triethyl citrate

### <Example 8>

Coated tablets were obtained in the same manner as in Example 1 except for use of the aqueous enteric coating liquid produced by changing the amount of purified water to 557.6 g and the concentration of the HPMCAS in the coating liquid to 14% by weight.

**Table 1**

| | Production of aqueous enteric coating liquid | | | | Permeability of First Liquid (%) | Tablet loss ratio (%) |
|---|---|---|---|---|---|---|
| | Neutralizer | Degree of neutralization (mol%) | Plasticizer ^{∗}1 | Aggregate | | |
| Example 1 | Aq. soln of ammonia | 20 | TEC | Not detected | 3.61 | 0 |
| Example 2 | Aq. soln of ammonia | 15 | TEC | Not detected | 4.16 | 0 |
| Example 3 | Aq. soln of ammonia | 5 | TEC | Not detected | 4.49 | 0 |
| Example 4 | Aq. soln of ammonia | 25 | TEC | Not detected | 4.99 | 0 |
| Example 5 | Aq. soln of sodium hydroxide | 20 | TEC | Not detected | 4.31 | 0 |
| Example 6 | Monoethanolamine | 20 | TEC | Not detected | 4.24 | 0 |
| Example 7 | Aq. soln of ammonia | 20 | TA | Not detected | 4.65 | 0 |
| Example 8 | Aq. soln of ammonia | 20 | TEC | Not detected | 3.81 | 0 |
| Comp. Ex. 1 | Aq. soln of ammonia | 20 | TEC | Detected | - | - |

| | | | | | | |
|---|---|---|---|---|---|---|
| ^{∗}1: As the plasticizer, TEC (triethyl citrate) or TA (triacetin) was used. | | | | | | |

## Claims

1. A method for producing an aqueous enteric coating liquid, comprising the steps of:
(i) partially neutralizing an aqueous suspension comprising a cellulosic enteric material with an aqueous alkali solution, and
(ii) mixing the partially neutralized aqueous suspension with a plasticizer,
wherein a degree of neutralization with the aqueous alkali solution is from 5 to 25 mol% of the carboxy groups of the cellulosic enteric material; and
wherein the cellulosic enteric material is a cellulose ester containing a carboxy group for preventing dissolution in an acidic liquid.

2. The method for producing an aqueous enteric coating liquid according to claim 1, wherein the cellulosic enteric material is selected from the group consisting of cellulose acetate phthalate, cellulose acetate trimellitate, cellulose acetate succinate, methyl cellulose phthalate, hydroxymethylethyl cellulose phthalate, hydroxypropylmethyl cellulose phthalate, hydroxypropylmethyl cellulose acetate succinate, hydroxypropylmethyl cellulose acetate maleate, hydroxypropylmethyl cellulose trimellitate.

3. The method for producing an aqueous enteric coating liquid according to claim 1 or claim 2, wherein the aqueous alkali solution is selected from the group consisting of respective aqueous solutions of ammonia, monoethanolamine and sodium hydroxide.

4. The method for producing an aqueous enteric coating liquid according to any one of claims 1, 2 or 3, wherein the plasticizer is selected form the group consisting of citric acid esters and glycerin fatty acid esters.

5. The method for producing an aqueous enteric coating liquid according to any one of claims 1 to 4, wherein the step of mixing comprises mixing the partially neutralized aqueous suspension with an excipient after, before or during mixing the partially neutralized aqueous suspension with the plasticizer.

6. The method for producing an aqueous enteric coating liquid according to claim 5, wherein the excipient is talc, titanium oxide or silicon dioxide.

7. A method for producing a solid preparation, comprising:
the method for producing an aqueous enteric coating liquid as claimed in any one of claims 1 to 6, and
a step of coating a core comprising a drug with the produced aqueous enteric coating liquid.

## Patentansprüche

1. Verfahren zum Herstellen einer wässrigen enterischen Beschichtungsflüssigkeit, umfassend die Schritte von:
(i) teilweisem Neutralisieren einer wässrigen Suspension, die ein enterisches Zellulosematerial mit einer wässrigen Alkalilösung umfasst und
(ii) Mischen der teilweise neutralisierten wässrigen Suspension mit einem Weichmacher, wobei ein Grad der Neutralisierung mit der wässrigen Alkalilösung 5 bis 25 Mol-% der Carboxygruppen des enterischen Zellulosematerials beträgt; und
wobei das enterische Zellulosematerial ein Zelluloseester ist, der eine Carboxygruppe zum Verhindern einer Auflösung in einer sauren Flüssigkeit enthält.

2. Verfahren zum Herstellen einer wässrigen enterischen Beschichtungsflüssigkeit nach Anspruch 1, wobei das enterische Zellulosematerial aus der Gruppe ausgewählt ist, die aus Zelluloseacetatphthalat, Zelluloseacetattrimellitat, Zelluloseacetatsuccinat, Methylzellulosephthalat, Hydroxymethylethylzellulosephthalat, Hydroxypropylmethylzellulosephthalat, Hydroxypropylmethylzelluloseacetatsuccinat, Hydroxypropylmethylzelluloseacetatmaleat, Hydroxypropylmethylzellulosetrimellitat besteht.

3. Verfahren zum Herstellen einer wässrigen enterischen Beschichtungsflüssigkeit nach Anspruch 1 oder Anspruch 2, wobei die wässrige Alkalilösung ausgewählt ist aus der Gruppe, die aus jeweiligen wässrigen Lösungen von Ammoniak, Monoethanolamin und Natriumhydroxid besteht.

4. Verfahren zum Herstellen einer wässrigen enterischen Beschichtungsflüssigkeit nach einem der Ansprüche 1, 2 oder 3, wobei der Weichmacher ausgewählt ist bilden die Gruppe, die aus Citronensäureestern und Glycerinfettsäureestern besteht.

5. Verfahren zum Herstellen einer wässrigen enterischen Beschichtungsflüssigkeit nach einem der Ansprüche 1 bis 4, wobei der Schritt des Mischens Mischen der teilweise neutralisierten wässrigen Suspension mit einem Hilfsstoff nach, vor oder während des Mischens der teilweise neutralisierten wässrigen Suspension mit dem Weichmacher umfasst.

6. Verfahren zum Herstellen einer wässrigen enterischen Beschichtungsflüssigkeit nach Anspruch 5, wobei der Hilfsstoff Talk, Titanoxid oder Siliciumdioxid ist.

7. Verfahren zum Herstellen einer festen Zubereitung, umfassend:
das Verfahren zum Herstellen einer wässrigen enterischen Beschichtungsflüssigkeit nach einem der Ansprüche 1 bis 6 und
einen Schritt eines Beschichtens eines Kerns, der ein Medikament mit der hergestellten wässrigen enterischen Beschichtungsflüssigkeit umfasst.

## Revendications

1. Procédé de production d'un liquide d'enrobage entérique aqueux, comprenant les étapes de :
(i) neutralisation partielle d'une suspension aqueuse comprenant un matériau entérique cellulosique avec une solution alcaline aqueuse, et
(ii) mélange de la suspension aqueuse partiellement neutralisée avec un plastifiant,
le degré de neutralisation avec la solution alcaline aqueuse représentant de 5 à 25 % en moles des groupes carboxy du matériau entérique cellulosique ; et
ledit matériau entérique cellulosique étant un ester de cellulose contenant un groupe carboxy pour empêcher la dissolution dans un liquide acide.

2. Procédé de production d'un liquide d'enrobage entérique aqueux selon la revendication 1, ledit matériau entérique cellulosique étant choisi dans le groupe constitué par le phtalate d'acétate de cellulose, le trimellitate d'acétate de cellulose, le succinate d'acétate de cellulose, le phtalate de méthylcellulose, le phtalate d'hydroxyméthyléthylcellulose, le phtalate d'hydroxypropylméthylcellulose, le succinate d'acétate d'hydroxypropylméthylcellulose, le maléate d'acétate d'hydroxypropylméthylcellulose, le trimellitate d'hydroxypropylméthylcellulose.

3. Procédé de production d'un liquide d'enrobage entérique aqueux selon la revendication 1 ou la revendication 2, ladite solution alcaline aqueuse étant choisie dans le groupe constitué par les solutions aqueuses respectives d'ammoniaque, de monoéthanolamine et d'hydroxyde de sodium.

4. Procédé de production d'un liquide d'enrobage entérique aqueux selon l'une quelconque des revendications 1, 2, ou 3, ledit plastifiant étant choisi forme le groupe constitué par les esters d'acide citrique et les esters d'acide gras de glycérine.

5. Procédé de production d'un liquide d'enrobage entérique aqueux selon l'une quelconque des revendications 1 à 4, ladite étape de mélange comprenant le mélange de la suspension aqueuse partiellement neutralisée avec un excipient, après, avant ou pendant le mélange de la suspension aqueuse partiellement neutralisée avec le plastifiant.

6. Procédé de production d'un liquide d'enrobage entérique aqueux selon la revendication 5, ledit excipient étant le talc, l'oxyde de titane ou le dioxyde de silicium.

7. Procédé de production d'une préparation solide, comprenant :
le procédé de production d'un liquide d'enrobage entérique aqueux selon l'une quelconque des revendications 1 à 6, et
une étape d'enrobage d'un cœur comprenant un médicament avec le liquide d'enrobage entérique aqueux produit.
